**Europäisches Patentamt**

**European Patent Office**

**Office européen des brevets**

(19)

(11) Numéro de publication: **0 233 108**
**A1**

(12) ## DEMANDE DE BREVET EUROPEEN

(21) Numéro de dépôt: **87400144.9**

(22) Date de dépôt: **22.01.87**

(51) Int. Cl.⁴: **A 61 B 5/00**, G 01 N 21/85

(30) Priorité: **24.01.86 FR 8601002**

(43) Date de publication de la demande: **19.08.87**
**Bulletin 87/34**

(84) Etats contractants désignés: **CH DE GB IT LI**

(71) Demandeur: **ETAT-FRANCAIS représenté par le DELEGUE GENERAL POUR L'ARMEMENT, Bureau des Brevets et Inventions de la Délégation Générale pour l'Armement 26, Boulevard Victor, F-75996 Paris Armées (FR)**

(72) Inventeur: **Testylier, Guy, 92, Avenue Jean-Jaurès, F-75019 Paris (FR)**
Inventeur: **Gourmelon, Patrick, 3, rue Léon Wirtzler, F-92330 Sceaux (FR)**

(54) **Spectrophotomètre pour dosage au sein d'un organisme vivant.**

(57) La présente invention concerne un spectrophotomètre, pour dosage dans un milieu biologique, par spectroscopie d'absorption, du type comprenant une source d'éclairement (1) monochromatique, des conducteurs (3, 5) de lumière pour la transmission d'un rayon d'éclairement vers le milieu à doser (4) et d'un rayon de mesure vers un photomultiplicateur (6) associé à des moyens de visualisation.

Il est caractérisé en ce que les conducteurs de lumière sont intégrés dans une microsonde, de diamètre compris entre 100 et 250 microns environ, présentant une première et une deuxième fibres optiques découplées optiquement par noircissement extérieur, pour la transmission respectivement du rayon d'éclairement et du rayon de mesure, et au moins un microcanal (7) en verre étiré, relié à un réservoir (10) contenant un réactif de dosage, ou une solution d'étalonnage, ou une solution ionique permettant la mesure de l'activité électrique du milieu exploré.

Application aux dosages in vivo, par exemple d'activités enzymatiques.

0233108

- 1 -

La présente invention concerne un spectrophotomètre permettant une mesure de densité optique ou de fluorescence au sein d'un milieu biologique. Le principe de cette spectroscopie d'absorption est fondé sur la mesure des variations de la lumière rétrodiffusée par le tissu lors d'une réaction colorimétrique initiée par injection d'un réactif.

Dans les applications médicales, on connaît, par exemple par le brevet FR-A-2 394 788, un spectophotomètre permettant la détermination de la coloration de la peau, et donc par une méthode externe, l'appréciation de l'état des surfaces de tissus irriguées par le sang.

Mais les dosages par spectrophotométrie sont des techniques anciennes et très répandues, s'appliquant à de nombreuses molécules d'intérêt biologique et à la mesure de l'activité d'un grand nombre d'enzymes. Cependant ils nécessitent dans la plupart des cas de tuer l'animal pour préveler le tissu intéressé, ou chez l'homme de pratiquer une biopsie. L'échantillon prélevé est ensuite traité, puis placé dans le spectrophotomètre.

Le fait de tuer l'animal et de prélever le tissu à analyser pose un très important problème méthodologique lorsque l'on veut étudier un système biologique dans son cadre physiologique et voir son évolution lorsqu'il est soumis à une substance pharmacologique ou à un toxique. Il est donc de première importance de développer des méthodes de dosage enzymologique ou de substances biologiques réalisables in situ et compatible avec la survie.

Les techniques concurrentes de dosage in vivo sont la polarographie et les techniques de push-pull canule. La polarographie est une technique fondée sur la mesure des potentiels d'oxydo-réduction de substances mais qui manque de spécificité. Le push-pull canule permet d'utiliser des méthodes de dosage spécifiques et précises, mais la petitesse des échantillons prélevés entraîne un très mauvais rapport signal sur bruit.

Cependant, on connaît par le brevet FR-A-2 451 185 un dispositif de détection in situ des variations de concentration des ions minéraux dans les tissus organiques à l'aide d'une microsonde comprenant trois microconduits convergents, dont un canal pour injecter une substance photoémettrice dans le milieu à doser, une fibre optique pour la transmission d'un rayon lumineux de mesure vers un photomultiplicateur, et une micropipette contenant une solution saline et une micro-électrode reliée à un dispositif de mesure de l'activité électrique des éléments cellulaires. Dans une variante, est envisagée la possibilité de réaliser des mesures d'absorption et de fluorescence en remplaçant le canal d'injection de la substance photo-émettrice par une fibre optique amenant un faisceau lumineux ultraviolet pour exciter la fluorescence de composés du tissu organique à doser. Mais dans tous les cas, le brevet précité ne décrit que l'utilisation de barreaux optiques en quartz étirés, dont l'extrémité pointue, forgée par étirage à chaud, est cassée sous contrôle microscopique. Or, les barreaux optiques étirés présentent d'importantes réflexions parasites au niveau du cône d'étirement empêchant le recueil de signaux interprétables.

La présente invention pallie les inconvénients relevés plus haut, et propose un spectrophotomètre miniaturisé permettant de réaliser in vivo, avec des résultats précis et reproductibles, les applications connues de la spectrophotométrie in vitro.

Pour ce faire, l'invention a pour objet un spectrophotomètre, pour dosage dans un milieu biologique par spectroscopie d'absorption, du type comprenant une source d'éclairement monochromatique, des conducteurs de lumière pour la transmission d'un rayon d'éclairement vers le milieu à doser et d'un rayon de mesure vers un photomultiplicateur associé à des moyens de mesure et/ou de visualisation, caractérisé en ce que les conducteurs de lumière sont intégrés dans une microsonde, de diamètre compris entre 100 et 250 microns environ, présentant une première et une deuxième fibres optiques découplées optiquement pour la transmission respectivement du rayon d'éclairement et du rayon de mesure, et au moins un microcanal en verre étiré.

Selon le cas, le microcanal est relié à un réservoir de réactif de dosage, ou est relié à un réservoir contenant une solution d'étalonnage ou contient une solution ionique permettant la mesure de l'activité électrique du milieu exploré.

D'autres avantages ressortiront de la description suivante d'un mode de réalisation, sans caractère limitatif, en référence au dessin annexé sur lequel :

- la figure 1 est une représentation schématique du dispositif selon l'invention, et

-les figures 2 et 3 représentent les résultats obtenus lors de l'application du spectrophotomètre selon l'invention à des mesures de l'acétycholinestérase cérébrale d'un rat, en utilisant le dosage colorimétrique d'ELLMAN.

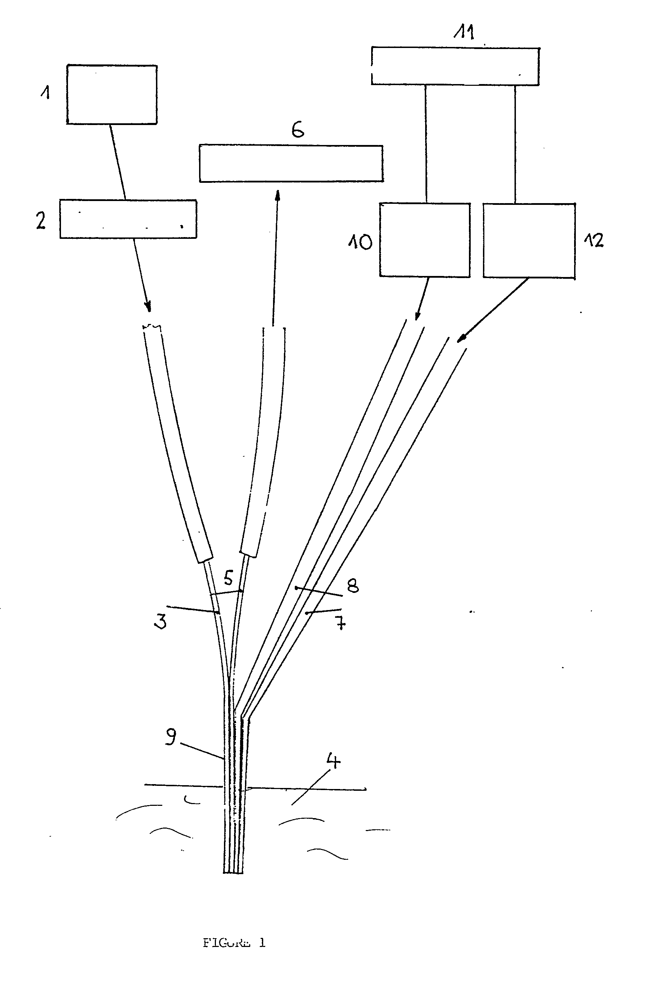

La figure 1 montre le spectrophotomètre comportant de façon connue un dispositif d'éclairement 1, par exemple une lampe à mercure à haute intensité, et un monochromateur 2, permettant de sélectionner une lumière dont la longueur d'onde se trouve dans la bande d'absorption de la substance à analyser. Un conducteur de lumière 3 transmet un rayon d'éclairement vers le milieu absorbant 4.

Pour la mesure de la quantité de lumière absorbée par le milieu, on compare les lumières rétrodiffusées en absence et en présence du corps absorbant, ou on compare les lumières rétrodiffusées à deux longueurs d'onde différentes, l'une située dans la bande d'absorption du corps, l'autre en dehors de cette bande.

De façon connue, la lumière rétrodiffusée est recueillie à l'aide d'un deuxième conducteur de lumière 5 qui est relié à un photomultiplicateur 6 pour être analysée et transmise à un système informatique de mesure et de visualisation.

Les conducteurs de lumière 3 et 5 sont constitués de deux fibres optiques de silice de diamètre voisin de 100 microns parfaitement cylindriques, ne produisant pas de réflexions parasites et de pertes de lumières.

Ces fibres sont découplées optiquement, par exemple, par noircissement extérieur avant d'être réunies par collage pour être intégrées dans une microsonde.

La comparaison des signaux obtenus dans les mêmes conditions avec une sonde constituée de barreaux optiques étirés tels que décrits dans le brevet FR-A-2 451 105 et la sonde à fibres optiques cylindriques noircies extérieurement selon l'invention montre ci-après les performances nécessaires à l'interprétation des signaux donnés par le photomultiplicateur.

L'intensité de lumière parasite qui était de 3000 mV dans l'antériorité, est ramenée à 10 mV; l'intensité des fluctuations passe de 50 mV dans l'antériorité à 2 mV dans le cas de l'invention. Enfin l'intensité de lumière réfléchie par un écran blanc, valant 60 mV dans le cas de l'utilisation de barreaux étirés, augmente notablement jusqu'à 600 mV dans le cas des fibres optiques cylindriques noircies.

A ces deux fibres optiques sont associés plusieurs microcanaux 7,8 en verre étiré et torsadé simultanément, présentant un diamètre d'environ 50 microns, qui permettent d'injecter soit le réactif de dosage, éventuellement des inhibiteurs de l'enzyme pour s'assurer de la spécificité du phénomène observé, soit des solutions de densité optique connue pour contrôler l'étalonnage.

Dans le cas de la fluorescence, il suffit de placer sur la fibre d'analyse un filtre qui arrête la lumière d'excitation rétrodiffusée.

Les fibres optiques et les microcanaux sont collés sous microscope avec une colle du type "cyanolyte", et l'ensemble est immobilisé dans une résine acrylique pour donner une microsonde 9 dont le diamètre est voisin de 250 microns.

La rigidité propre de cette sonde 9, de longueur voisine de 25 mm, permet de l'implanter telle quelle dans un tissu mou comme le cerveau; dans d'autres tissus, il suffit de la placer dans une microcanule métallique.

On réalise à l'aide de ce dispositif des mesures de l'acétylcholinestérase cérébrale en utilisant le dosage colorimétrique d'ELLMAN.

Le réactif d'ELLMAN est constitué d'acétylthiocholine qui, hydrolysée par l'enzyme, forme avec l'acide dithio-bis-nitrobenzoïque (DTNB) un colorant jaune absorbant la lumière vers 0,410 microns. Ce réactif est injecté, à partir d'un réservoir 10 dans l'un des microcanaux grâce à une pompe pneumatique 11 qui fournit des impulsions de pression d'une durée variable, par exemple 500 ms sous $2.10^5$ Pa.

Le deuxième canal 8 sert à injecter, à partir d'un réservoir 12, un inhibiteur organophosphoré de l'acétylcholinestérase, par exemple du MPT (O-éthyl-S-(2-diisopropylamino-éthyl)-méthyl-phosphonothioate). Il permet de s'assurer que les variations de lumière rétrodiffusée observées sont effectivement dues au fonctionnement de l'enzyme.

Après amplification du signal issu du photomultiplicateur, celui-ci est transmis à un convertisseur analogique-digital connecté à un calculateur, qui permet ensuite d'obtenir les résultats sous forme graphique.

On décrit ci-après des résultats obtenus lorsque la sonde est implantée dans le système nerveux central au niveau du striatum chez un rat anesthésié.

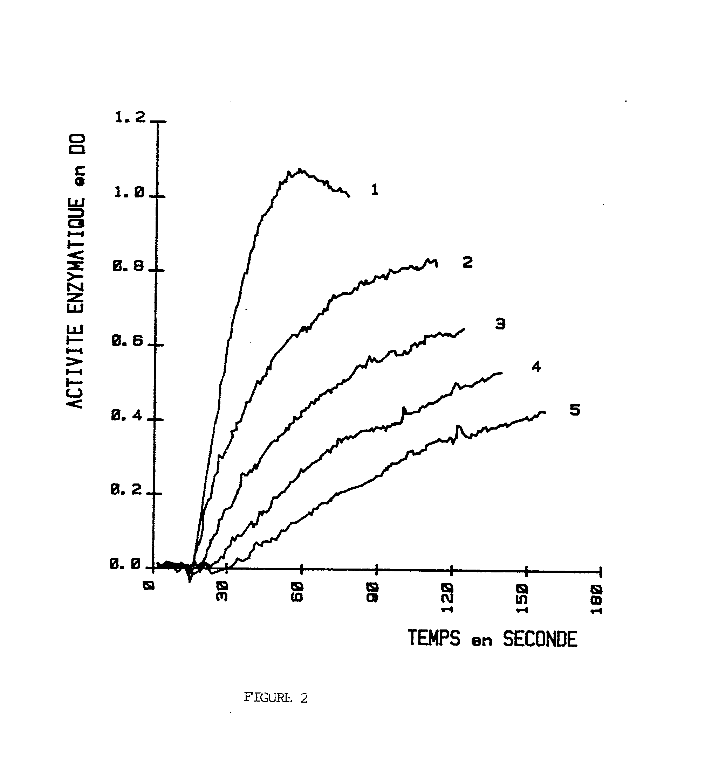

La figure 2 montre l'activité de l'acétylcholinestérase dans le striatum de cerveau de rat. La mesure est initialisée par injection de 75 nl de réactif d'ELLMAN :

1 sans MPT

2 après injection de 9 fmole de MPT ($9.10^{-15}$ mole)

3 18 fmole de MPT

4 27 fmole de MPT

5 36 fmole de MPT.

On notera le très bon rapport signal sur bruit qui permet de penser qu'une miniaturisation encore plus grande de la sonde est possible.

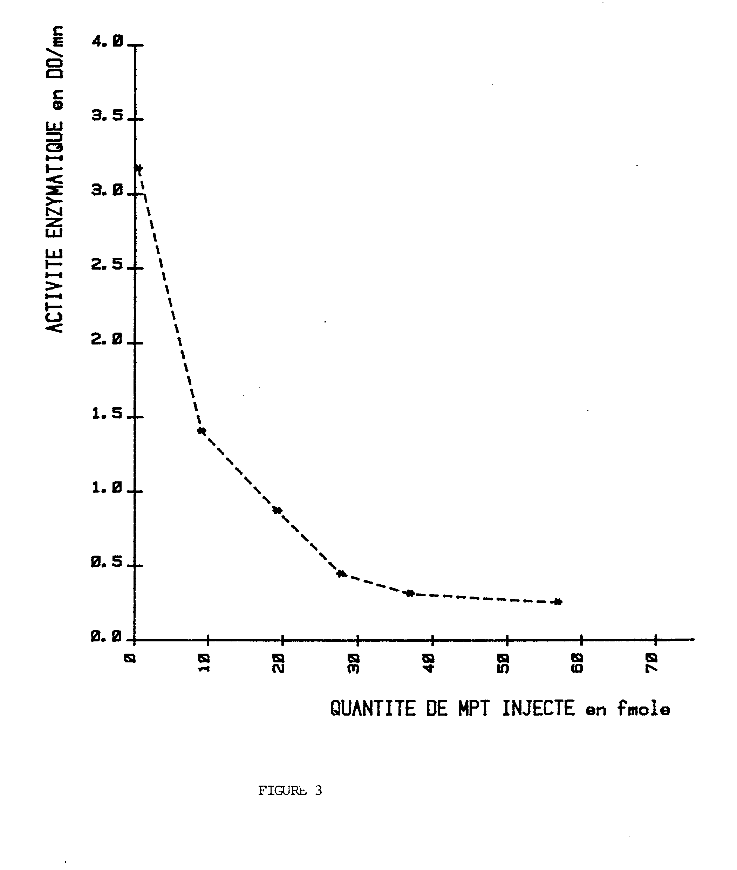

La figure 3 montre la variation de l'activité de l'acétylcholinestérase, par mesure de la densité optique par minute, en fonction de la quantité de MPT injectée sur le site de mesure. On mesure alors la quantité d'enzyme vue par la sonde et qui a donc été neutralisée par l'inhibiteur.

De multiples dosages successifs ont pu être pratiqués pendant plusieurs heures sur des rats anesthésiés ou chroniques sans aucun effet secondaire cliniquement décelable les jours suivants.

- 6 -

D'autres applications du spectrophotomètre miniaturisé selon l'invention sont possibles.

De nombreuses activités enzymatiques peuvent être dosées par des méthodes colorimétriques, si elles remplissent les conditions suivantes : la réaction colorée doit être réalisable dans des conditions physiologiques de température et de pH, le colorant formé doit de préférence avoir son spectre dans le visible ou le proche ultraviolet pour ne pas être noyé dans l'absorption des protéines, et le dosage doit être réalisable sur un enzyme au sein d'un homogénat. Une cartographie précise de l'enzyme peut alors être réalisée ainsi que toute étude pharmacologique affectant cet enzyme.

Par ailleurs les mesures effectuées à différentes longueurs d'onde permettent de reconstituer un spectre d'absorption après correction du spectre de la lampe. Ceci permet d'indentifier et de doser la substance absorbante.

De plus, de nombreuses méthodes de dosage utilisant la fluorescence propre ou induite des substances à doser peuvent être mises en oeuvre avec le dispositif selon l'invention dans le cas de fluorescence induite si la réaction est compatible avec la survie de l'animal, si les longueurs d'onde d'excitation et de fluorescence sont en dehors de celle des protéines et des acides nucléiques, et si le dosage ne nécessite pas de purification préalable.

Un des microcanaux peut être utilisé pour le recueil de l'activité électrique unitaire neuronale permettant d'analyser les relations temporelles entre le signal électrophysiologique et l'activité biochimique.

- 7 -

REVENDICATIONS

1 - Spectrophotomètre, pour dosage dans un milieu biologique par spectroscopie d'absorption, du type comprenant une source d'éclairement (1) monochromatique, des conducteurs (3,5) de lumière pour la transmission d'un rayon d'éclairement vers le milieu à doser (4) et d'un rayon de mesure vers un photomultiplicateur (6) associé à des moyens de visualisation, caractérisé en ce que les conducteurs de lumière sont intégrés dans une microsonde, de diamètre compris entre 100 et 250 microns environ, présentant une première et une deuxième fibres optiques découplées optiquement pour la transmission respectivement du rayon d'éclairement et du rayon de mesure, et au moins un microcanal (7) en verre étiré.

2 - Spectrophotomètre selon la revendication 1, caractérisé en ce que les fibres optiques découplées optiquement par noircissement extérieur.

3 - Spectrophotomètre selon la revendication 1, caractérisé en ce que le microcanal est relié à un réservoir de réactif de dosage.

4 - Spectrophotomètre selon la revendication 1, caractérisé en ce que le microcanal est relié à un réservoir contenant une solution d'étalonnage.

5 - Spectrophotomètre selon la revendication 1, caractérisé en ce que le microcanal contient une solution ionique permettant la mesure de l'activité électrique du milieu exploré.

FIGURE 1

FIGURE 2

PL 3 / 3.

FIGURE 3

Office européen
des brevets

RAPPORT DE RECHERCHE EUROPEENNE

0233108
Numéro de la demande

EP 87 40 0144

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y,D | FR-A-2 451 185 (E. LABEYRIE) <br> * Page 1; figure 1 * | 1,5 | A 61 B 5/00 <br> G 01 N 21/85 |
| Y | DE-A-2 053 301 (FRITZ HELLIGE & CO., GmbH) <br> * Pages 2,3; figures 1-3 * | 1 | |
| A | GB-A-2 136 120 (SHIONOGI SEIYAKU K.K.) <br> * Page 4; figures 3-4 * | 1,2 | |
| A | EP-A-0 093 927 (Fa. CARL ZEISS) <br> * Page 5; figures 2,3,5 * | 1 | |
| A | ANALYTICAL CHEMISTRY, vol. 56, no. 12, octobre 1984, pages 2246-2249, American Chemical Society, Easton, Pennsylvania, US; J.T: COLEMAN et al.: "Fiber optic based sensor for bioanalytical absorbance measurements" <br> * Pages 2246-2247; figure 1 * | 1 | **DOMAINES TECHNIQUES RECHERCHES (Int. Cl.4)** <br><br> A 61 B 5/00 <br> G 01 N 21/77 <br> G 01 N 21/85 |

Le présent rapport de recherche a été établi pour toutes les revendications

| Lieu de la recherche | Date d'achèvement de la recherche | Examinateur |
|---|---|---|
| LA HAYE | 04-05-1987 | BOEHM CH.E.D. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82